# EUROPEAN PATENT APPLICATION

(11) **EP 3 040 422 A1**
(43) Date of publication of application: **06.07.2016**
(21) Application number: 14841107.7
(22) Date of filing: 28.08.2014
(51) Int. Cl.: C12Q 1/68, C12N 15/09

(54) **METHOD FOR DETECTING PREDISPOSITION FOR HEPATITIS B TO BECOME CHRONIC**

(30) Priority: 30.08.2013 JP 2013179634
(71) Applicant: The University of Tokyo, Tokyo 113-8654 (JP); National Center for Global Health and Medicine, Tokyo 162-8655 (JP)
(72) Inventor: TOKUNAGA Katsushi, Tokyo 113-8654 (JP); SAWAI Hiromi, Tokyo 113-8654 (JP); MIZOKAMI Masashi, Ichikawa-shi Chiba 272-8516 (JP); NISHIDA Nao, Ichikawa-shi Chiba 272-8516 (JP)
(74) Representative: Desaix, Anne
(86) International application number: PCT/JP2014/072649
(87) International publication number: WO 2015/030142

(57) **Abstract**

An object of the present invention is to provide a method for detecting predisposition for chronicity of hepatitis B and/or the pathological progress, including an allele associated with chronicity of hepatitis B and/or the pathological progress. The above-described object is achieved by providing a method that includes a process of identifying alleles which are susceptible and resistant to chronicity of hepatitis B and/or the pathological progress in which a difference between a chronic hepatitis B patient group and a healthy control group is accurately reflected and comparing the alleles with base sequences or amino acid sequences corresponding to the alleles in a specimen; a process of analyzing whether bases of sites corresponding to the alleles in the base sequences of the specimen match bases of the alleles; and a process of specifying whether the hepatitis B of the specimen becomes chronic and/or the pathology is progressed, a method of performing inspection using the method, a reagent used for the method, and a test kit including the reagent.

## Description

### Technical Field

The present invention relates to a method for detecting predisposition of chronicity of hepatitis B and/or the pathological progress, including alleles associated with chronicity of hepatitis B and/or the pathological progress, a method for inspecting chronic hepatitis B or the pathological progress, a reagent for detecting predisposition of chronicity of hepatitis B and/or the pathological progress, and a test kit which includes the reagent for detecting chronicity of hepatitis B and/or the pathological progress.

### Background Art

It is said that people corresponding to 1/3 of the world population are infected with hepatitis B virus (HBV). Hepatitis B is largely divided into transient infection which is ceased as a transient one and chronic hepatitis. In a case of acute hepatitis, symptoms appear after an incubation period of 1 month to 6 months from infection and enter a recovery process in a few weeks. However, 1% to 2% of the patients who have developed acute hepatitis are at risk of developing fulminant hepatitis and 70% to 80% of people who develop fulminant hepatitis die.

In addition, a person infected with HBV is turned into a carrier by HBV settling into the liver for over 6 months without being expelled from the body. 80% to 90% of carriers pass through an asymptomatic period, a transient hepatitis period, and a hepatitis calm period and then remain as asymptomatic carriers. However, 10% to 20% of the carriers transition to carriers with chronic hepatitis and some of the carriers transition to carriers with cirrhosis or liver cancer. The chronic hepatitis B carriers are largely distributed in Southeast Asia and the Eastern Pacific Ocean region. Particularly, it is said that there are 1500000 people infected with hepatitis B in Japan.

In this manner, the progress after HBV infection is diverse and genetic factors of the virus side mainly associated with chronic hepatitis and liver cancer have been investigated. However, in recent years, the search for genetic factors of the host side has advanced and new genetic factors associated with chronic hepatitis B (CHB) have been reported by Genome Wide Association Study (GWAS) using Asian samples including the Japanese. It is suggested that HLA-DPA1 and HLA-DPB1 are associated with HBV persistent infection or viral clearance (NPLs 1 and 2), and suggested that HLA-DR and HLA-DQ having linkage disequilibrium are associated therewith (NPL 3). However, in the analysis using the GWAS, strong genetic factors other than those associated with chronicity have not been found. Moreover, several host genetic factors associated with cancer have been reported from China by the recent GWAS (NPLs 4 and 5).

### Citation List

### Non-Patent Literature

[NPL 1] Kamatani Y, Wattanapokayakit S, Ochi H, Kawaguchi T, Takahashi A, et al. (2009) A genome-wide association study identifies variants in the HLA-DP locus associated with chronic hepatitis B in Asians. Nat Genet 41: 591 to 595.
[NPL 2] Nishida N, et.al., Genome-Wide Association Study Confirming Association of HLA-DP with Protection against Chronic Hepatitis B and Viral Clearance in Japanese and Korean Plos ONE June 2012 vol. 7, Issue 5, e39175
[NPL 3] Mbarek H, Ochi H, Urabe Y, Kumar V, Kubo M, et al. (2011) A genome-wide association study of chronic hepatitis B identified novel risk locus in a Japanese population. Hum Mol Genet 20: 3884 to 3892
[NPL 4] Li S, Qian J, Yang Y, Zhao W, Dai J, et al. (2012) GWAS Identifies Novel Susceptibility Loci on 6p21.32 and 21q21.3 for Hepatocellular Carcinoma in Chronic Hepatitis B Virus Carriers. PloS Genet 7(5): e39175
[NPL 5] Jiang DK, Sun J, Cao G, Liu Y, Lin D, et al. (2013) Genetic variants in STAT4 and HLA-DQ genes confer risk of hepatitis B virus-related hepatocellular carcinoma. Nat Genet 45: 72-5

### Summary of Invention

### Technical Problem

In regard to HLA-DP, there is a report that the allele frequency is researched by comparing a chronic hepatitis B patient group with a comparison control group (NPL 1). However, since a non-hepatitis patient group is used as the comparison control group in the research on the allele frequency, there is a problem in that it is unclear whether a difference between the chronic hepatitis B patient group and the healthy control group is reflected in the experiment results. In addition, the relation between the pathological progress and the HLA-DPB allele has not been investigated.

Here, an object of the present invention is to provide a method for detecting genetic predisposition of chronicity of hepatitis B and/or the pathological progress, including alleles associated with chronicity of hepatitis B and/or the pathological progress by means of performing analysis using a healthy control group, an HBV patient group, a chronic hepatitis B group, and a hepatitis B pathology development group (cirrhosis or liver cancer) and identifying alleles which are susceptible and resistant to chronic hepatitis B and the pathological progress, a method for inspecting chronic hepatitis B, liver cirrhosis, or liver cancer, a reagent for detecting predisposition of chronicity of hepatitis B and/or the pathological progress, and a test kit which includes the reagent for detecting chronicity of hepatitis B and/or the pathological progress. Solution to Problem

The present inventors found alleles associated with chronicity of hepatitis B by performing typing associated with HLA-DP using a healthy control group, an HBV patient group, a chronic hepatitis B group, and a hepatitis B pathology development group (cirrhosis or liver cancer). In this manner, a method of detecting predisposition of chronicity of hepatitis B and the pathological progress, a method of inspecting chronic hepatitis B, cirrhosis, or liver cancer, a reagent for detecting predisposition of chronicity of hepatitis B and the pathological progress, and a test kit which includes the reagent for detecting chronicity of hepatitis B and the pathological progress are constructed using the alleles.

That is, the present invention is as follows.
(1) A method for detecting predisposition for chronicity of hepatitis B and/or the pathological progress, the method including: a) a process of comparing alleles associated with chronicity of hepatitis B and/or the pathological progress with base sequences or amino acid sequences corresponding to the alleles in a specimen; b) a process of analyzing whether the bases or amino acid residues of sites corresponding to the alleles of the specimen match bases or amino acid residues of the alleles; and c) a process of specifying whether the hepatitis B of the specimen becomes chronic and/or the pathology is progressed.
(2) The method according to (1), in which the alleles associated with chronicity of hepatitis B and/or the pathological progress are susceptible or resistant to chronicity of hepatitis B and/or the pathological progress.
(3) The method according to (2), in which a combination of the alleles associated with chronicity of hepatitis B and/or the pathological progress is any of a combination of alleles only having the susceptibility, a combination of alleles only having the resistivity, and a combination of alleles having the susceptibility and alleles having the resistivity.
(4) The method according to (2) or (3), in which the alleles which are susceptible to chronicity of the hepatitis B are HLA-DPB1*05:01 and HLA-DPB1*09:01, the alleles which are resistant to chronicity of the hepatitis B are HLA-DPB1*04:02, HLA-DPB1*04:01, and HLA-DPB1*02:01, and the alleles which are resistant to the pathological progress of the chronic hepatitis B are HLA-DPB1*02:01.
(5) The method according to any one of (2) to (4), in which the combination of the alleles which are resistant to the chronicity ofthe hepatitis B is HLA-DPB1*02:01, HLA-DPB1*04:01 or HLA-DPB1*04:02, and HLA-DPB1*05:01 or HLA-DPB1*09:01.
(6) A method of inspecting chronicity of hepatitis B and/or the pathological progress using the method according to any one of (1) to (5).
(7) A reagent for detecting predisposition of chronicity of hepatitis B and/or the pathological progress, the reagent including a primer which detects alleles associated with chronicity of hepatitis B and/or the pathological progress.
(8) The reagent according to (7), in which the alleles associated with chronicity of the hepatitis B and/or the pathological progress are susceptible or resistant to chronicity of hepatitis B and/or the pathological progress.
(9) The reagent according to (8), in which a combination of the alleles associated with chronicity of hepatitis B and/or the pathological progress is any of a combination of alleles only having the susceptibility, a combination of alleles only having the resistivity, and a combination of alleles having the susceptibility and alleles having the resistivity.
(10) The reagent according to (8) or (9), in which the alleles which are susceptible to chronicity of the hepatitis B are HLA-DPB1*05:01 and HLA-DPB1*09:01, the alleles which are resistant to chronicity of the hepatitis B are HLA-DPB1*04:02, HLA-DPB1*04:01, and HLA-DPB1*02:01, and the alleles which are resistant to the pathological progress of the chronic hepatitis B are HLA-DPB1*02:01.
(11) The reagent according to any one of (8) to (10), in which the combination ofthe alleles which are resistant to the chronicity of the hepatitis B is HLA-DPB1*02:01, HLA-DPB1*04:01 or HLA-DPB1*04:02, and HLA-DPB1*05:01 or HLA-DPB1*09:01.
(12) A kit comprising a reagent which contains alleles associated with chronicity of hepatitis B and/or the pathological progress according to any one of (7) to (11) and is used for detecting predisposition of chronicity of hepatitis B and/or the pathological progress.

### Advantageous Effects of Invention

By analyzing alleles associated with chronicity of hepatitis B of the present invention and/or the pathological progress with respect to an HBV patient group, it is possible to elucidate a molecular mechanism of chronicity of hepatitis B and/or the pathological progress and to identify target candidate molecules of drug development. In addition, by analyzing the alleles with respect to HBV carriers, there is an advantage that the carriers can be classified into a group in which chronicity and the pathological progress are likely to occur and a group in which chronicity and the pathological progress are unlikely to occur so that information useful to determine the subsequent treatment policy can be provided. Accordingly, it is possible to prevent chronicity of hepatitis B and/or the pathological progress, prevent the progression, and perform appropriate treatment.

Moreover, in regard to the genetic predisposition of chronicity of hepatitis B and the pathological progress, it is not clear whether there is a similar tendency between Asians including the Japanese and Westerners. Using the alleles associated with chronicity of hepatitis B of the present invention and/or the pathological progress, determination of treatment policy of chronic hepatitis B specialized for Asians including the Japanese and/or the pathological progress and identification of target candidate molecules of drug development become possible.

Moreover, there is an effect that a test kit with higher precision, which includes the alleles and SNP of other immune-related genes can be developed.

### Brief Description of Drawings

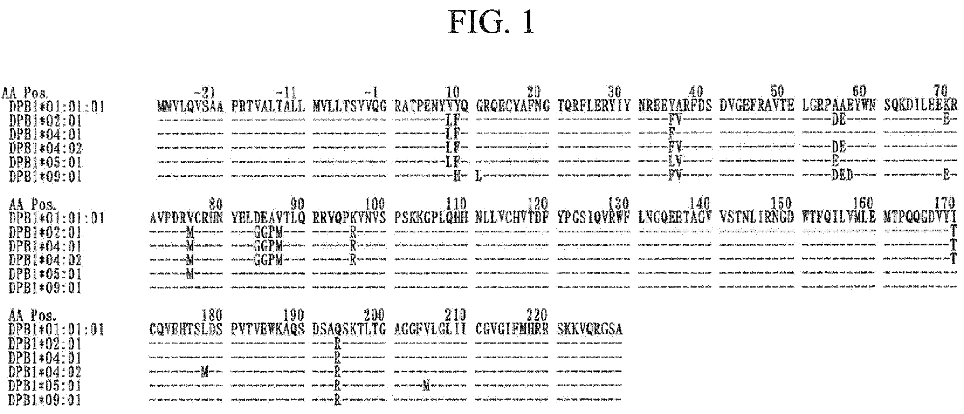

FIG. 1 shows alignment of alleles of the present invention and the amino acid sequence of DPB 1*01:01:01.

### Description of Embodiments

The present invention relates to a method for detecting genetic predisposition of chronicity of hepatitis B and/or the pathological progress, including alleles associated with chronicity of hepatitis B and/or the pathological progress, a method for inspecting chronic hepatitis B or the pathological progress, a reagent for detecting predisposition of chronicity of hepatitis B and/or the pathological progress, and a test kit which includes the reagent for detecting chronicity of hepatitis B and/or the pathological progress. The present invention will be described below.

### (1) Alleles associated with chronicity of hepatitis B according to present invention and/or pathological progress

The present invention relates to alleles associated with chronicity of hepatitis B and/or the pathological progress. The alleles associated with the chronicity of hepatitis B and the pathological progress are susceptible (hepatitis B is likely to be chronic and the pathology is likely to progress) or resistant (hepatitis B is unlikely to be chronic and the pathology is unlikely to progress) to chronicity of hepatitis B and the pathological progress.

In the present invention, the "chronicity of hepatitis B" indicates a state of persistant infection with HBV. As the onset factors, a case of maternal infection (vertical infection) from a person persistently infected with HBV; a case in which the blood or the body fluid of a person persistently infected with HBV enters the body due to medical practice in infancy; a case in which HBV is unable to be eliminated from the body and persistent infection is caused as a result of infection with HBV during the use of an immunosuppressive agent or an anti-cancer agent that reduces immunity of the body; and a case in which, in recent years, a healthy person is infected with Western type or Asian and African type of alien HBV species which is a genotype A are exemplified. In this manner, when infected with HBV, 80% to 90% of the infected people become asymptomatic carriers and 10% to 20% thereof transition to carriers with chronic hepatitis. Further, some of the carriers transition to carriers with cirrhosis or liver cancer. Here, a group that undergoes transition from chronicity of HBV to cirrhosis or liver cancer is referred to as the "pathological progress of hepatitis B" of the present invention. The "cirrhosis" indicates a state in which fibers which are generated when the liver damaged by hepatitis virus infection is repaired have spread to the liver, and this becomes a factor of the occurrence of ascites or esophageal varices due to the liver being hard or the occurrence of hepatic encephalopathy or jaundice due to degradation of the liver function. The "liver cancer" indicates liver cell cancer that is caused by the infection with the hepatitis virus.

The search for the alleles associated with chronicity of hepatitis B and/or the pathological progress is performed by preparing genomic DNA from biological samples collected from chronic hepatitis B patients, cirrhosis or liver cancer patients, and healthy controls and analyzing the gene sequences according to the direct sequencing method or the like. Since new alleles obtained in the above-described manner are found from chronic hepatitis B patients or cirrhosis or liver cancer patients, the new alleles are promising candidates as the alleles of the present invention which are highly associated with chronicity of hepatitis B and/or the pathological progress. The association of the alleles selected in the above-described manner with chronicity of hepatitis B and/or the pathological progress can be confirmed by performing a statistical test. For example, the incidence of the candidate allele in a group of people infected with chronic hepatitis B and a healthy control group is respectively calculated and the association of the candidate allele with the chronicity of hepatitis B is statistically tested. The test can be performed according to a statistically appropriate method such as χ2 test or Fischer's exact test, and correction in a significant level may be performed as needed.

The method of detecting the alleles is not particularly limited and can be selected from methods known by those skilled in the art. For example, the method can be selected from known typing methods such as a TaqMan PCR method, a MALDI-TOF/MS method, an allele-specific oligonucleotide (ASO) method, a direct sequencing method, an RFLP method, an invader method, a TGGE method, a DGGE method, an MutY enzymatic method, microarray, a protein truncation test (PTT) method, and a Snipper method according to the purposes thereof. Although many methods apply the PCR method, there are methods which do not depend on the PCR method.

The typing of HLA-DPB1 was performed, by the present inventors, on 489 specimens of the HBV patient group and 467 specimens of the healthy control group of Japanese people and 340 specimens of the HBV patient group and 140 specimens of the healthy control group of Korean people, and HLA-DPB1*05:01 and HLA-DPB1*09:01 were identified as alleles which are susceptible to chronicity of hepatitis B and HLA-DPB1*04:02, HLA-DPB1*04:01, and HLA-DPB1*02:01 were identified as alleles which are resistant to chronicity of hepatitis B. In addition, HLA-DPB1*02:01 was identified as the allele which is resistant to the pathological progress of chronic hepatitis B. The HLA is a major histocompatibility complex (MHC) of a human and is membrane protein that is bonded to exogenous antigenic peptides such as grafts, a bacteria, or viruses to be presented to T cells. It is known that a large amount of alleles are present in HLA and the information is described in HLA nomenclature (http://hla.alleles.org/announcement.html). Further, the notation of the alleles of the present specification or polymorphism is based on a notation method described in HLA nomenclature.

As the sequence data related to the alleles, data registered in the database such as GenBank (NIH genetic sequence database) or DNA data bank of Japan (DDBJ) may be used. Here, for example, cDNA of HLA-DPB1*05:01 is the 917 base and registered as AY804138 (SEQ ID NO: 1) in GenBank and the amino acid sequence is the 258 residue and is registered as AAW78743 (SEQ ID NO: 2). Further, cDNA of HLA-DPB1*09:01 is the 907 base and registered as AY804139 (SEQ ID NO: 3) in GenBank and the amino acid sequence is the 258 residue and is registered as AAW78744 (SEQ ID NO: 4). Further, cDNA of HLA-DPB1 *04:02 is the 917 base and registered as AY804137 (SEQ ID NO: 5) in GenBank and the amino acid sequence is the 258 residue and is registered as AAW78742 (SEQ ID NO: 6). Further, cDNA of HLA-DPB1*04:01 is the 883 base and registered as AY804136 (SEQ ID NO: 7) in GenBank and the amino acid sequence is the 258 residue and is registered as AAW78741 (SEQ ID NO: 8). Further, cDNA of HLA-DPB1*02:01 is the 908 base and registered as AY804134 (SEQ ID NO: 9) in GenBank and the amino acid sequence is the 258 residue and is registered as AAW78739 (SEQ ID NO: 10). The amino acid sequence of DPB1*01:01:01 is shown in SEQ ID NO: 11 (AAW78748) and FIG. 1 shows alignment of the amino acid sequence and the alleles of the present invention.

Moreover, the above-described alleles HLA-DPB1*05:01, HLA-DPB1*09:01, HLA-DPB1*04:02, HLA-DPB1*04:01, and HLA-DPB1*02:01 are referred to as "the alleles associated with chronicity of hepatitis B of the presenet invention and/or the pathological progress," "the alleles associated with chronicity of hepatitis B of the present invention," "the alleles according to the present invention," or polymorphism or SNP instead of alleles in some cases.

The alleles used in the present invetnion include single-stranded and double-stranded DNA and an RNA complement thereof, and may be naturally generated or artificially produced. Examples of DNA include genomic DNA, cDNA corresponding to the genomic DNA, chemically synthesized DNA, DNA which is amplified by PCR, a combination of these, and a hybrid of DNA and RNA, but are not limited to these. In addition, the polynucleotide in the present invention indicates two or more nucleotides being bonded to each other and those generally with the length of oligonucleotides are included. Further, the polynucleotide of the present invention may be DNA or RNA.

These base sequences can be obtained from a cDNA library and a genomic library using a probe, according to a known hybridization method such as colony hybridization, plaque hybridization, or Southern blotting, after the probe is prepared using a suitable fragment according to a method known by those skilled in the art.

Specific procedures of the hybridization method can be referred to in "Molecular Cloning, A laboratory Manual 3rd ed." (Cold Spring Harbor Press (2001): particularly Section 6 and 7), "Current Protocols in Molecular Biology" (John Wiley & Sons (1987 to 1997); particularly Section 6.3 and 6.4), and "DNA Cloning 1: Core Techniques, A Practical Approach 2nd ed." (Oxford University (1995); the hybridization conditions can be particularly referred to in Section 2.10).

### (2) Method of detecting predisposition of chronicity of hepatitis B and/or pathological progress using alleles according to present invention

The present invention relates to a method (typing method) of detecting predisposition of chronicity of hepatitis B and/or the pathological progress using the alleles (in the description below, also referred to as "polymorphism" or "SNP" in some cases) associated with the chronicity of hepatitis B and/or the pathological progress of the present invention. Specifically, the method includes the following processes: a) a process of comparing alleles associated with chronicity of hepatitis B and/or the pathological progress with base sequences or amino acid sequences corresponding to the alleles in a specimen; b) a process of analyzing whether bases or amino acid residues of sites corresponding to the alleles of the specimen match bases or amino acid residues of the alleles; and c) a process of specifying whether the hepatitis B of the specimen becomes chronic and/or the pathology is progressed. The above-described method of the present invention is based on the knowledge that the alleles susceptible to chronicity of hepatitis B are HLA-DPB1*05:01 and HLA-DPB1*09:01, the alleles resistant to chronicity of hepatitis B are HLA-DPB1*04:02, HLA-DPB1*04:0̇1, and HLA-DPB1*02:01, and the allele resistant to the pathological progress of chronic hepatitis B is HLA-DPB1*02:01, as described in "(1) the alleles associated with chronicity of hepatitis B of the present invention and/or the pathological progress." The method will be described below in detail.

"The a) process of comparing alleles associated with chronicity of hepatitis B and/or the pathological progress with base sequences or amino acid sequences corresponding to the alleles in a specimen" is a process of detecting the base sequences shown in SEQ ID NOS: 1 to 10 and alleles of the amino acid sequences.

In the present invention, the detection of alleles can be performed in the gene level or in the protein level. For example, genomic DNA or mRNA is prepared from a specimen and alleles associated with chronicity of hepatitis B of the present invention in the genomic DNA or mRNA can be detected based on the base sequences. Further, human HLA-DP molecule protein is prepared from the specimen and alleles of HLA-DPB1*09:01, HLA-DPB1*05:01, HLA-DPB1*04:02, HLA-DPB1*04:01, and/or HLA-DPB1*02:01 in DP molecules can be detected using, for example, an antibody. These methods will be simply described below.

### (2-1) Preparation of genomic DNA or mRNA from specimen

As the samples used in the method of the present invention, genomic DNA or mRNA prepared according to a method known by those skilled in the art can be used based on biological samples collected from a specimen to be inspected to determine whether the specimen is susceptible or resistant to chronicity of hepatitis B and/or the pathological progress. As the biological samples collected from the specimen used in the method of the present invention, cells of the specimen or cells scraped from, for example, the tissues, hair, feces, urine, saliva, cells, or nasal mucosa can be used, but the samples are not limited thereto.

Genomic DNA can be prepared according to any known methods, and examples of the methods include a phenol/chloroform method and a cetyl trimethyl ammonium bromide (CTAB) method. Further, mRNA can be prepared according to any known methods, and examples of the methods include a guanidine isothiocyanate method. Commercially available kits may be used for the preparation of genomic DNA or mRNA. As the kits, a Wizard Genomic DNA Purification Kit (Promega) and a NucleoTrap (registered trademark) mRNA Kit (Clontech) may be respectively used for preparation of genomic DNA and mRNA. In addition, cDNA may be synthesized from mRNA to detect the alleles described below. Further, any known methods in the art may be used for the method of synthesizing cDNA. For example, cDNA can be synthesized from RNA by a reverse transcriptase-polymerase chain reaction (RT-PCR) using a random primer or a poly T primer.

### (2-2) Detection of alleles

The alleles of HLA-DPB1*09:01, HLA-DPB1*05:01, HLA-DPB1*04:02, HLA-DPB1*04:01, and/or HLA-DPB1*02:01 in the genomic DNA or mRNA prepared in the above-described manner can be detected using any known genetic polymorphism detection means in the art. Examples of the detection method include methods using a direct sequencing method, a polymerase chain reaction (PCR), restriction fragment length polymorphism (RFLP), a hybridization method, a primer extension reaction, or mass spectrometry, but are not limited to these. In this case, since multiple polymorphic sites are present particularly in the second exon of the HLA-DRB1 gene, the multiple polymorphisms need to be determined so as to detect these alleles. Hereinafter, the method will be described.

### (2-2-1) Direct sequencing method

In the present invention, the alleles of HLA-DPB1 *09:01, HLA-DPB1 *05:01, HLA-DPB1*04:02, HLA-DPB1*04:01, and/or HLA-DPB1*02:01 can be detected by the direct sequencing method using cDNA derived from genomic DNA or mRNA. The direct sequencing method is performed by preparing cDNA from the genomic DNA or mRNA prepared in the above-described manner, cloning a region, including the alleles of HLA-DPB1*09:01, HLA-DPB1*05:01, HLA-DPB1*04:02, HLA-DPB1*04:01, and/or HLA-DPB1*02:01 which are detection targets, in a vector or amplifying the same by PCR, and determining the base sequences of the region. In the cloning method, the region can be cloned by performing screening from the cDNA library using a suitable probe. Further, the region can be cloned by performing amplification through a PCR reaction using a suitable primer and linking the region to a suitable vector. Further, the region can be subcloned in another vector, but the cloning is not limited thereto. Examples of the vector include commercially available plasmid vectors such as pBlue-Script (trademark) SK (+) (Stratagene Corp.), pGEM-T (Promega Corporation), pAmp (TM: Gibco-BRL), p-Direct (Clontech), and pCR2.1-TOPO (Invitrogene); viral vectors; artificial chromosome vectors; and cosmid vectors. Any known methods can be used for determination of the base sequences, and examples thereof include a manual sequencing method using radioactive marker nucleotides and an automatic sequencing method using a dye-terminator, but the examples are not limited to these. It is determined whether the specimen has the sequences corresponding to the alleles of HLA-DPB1*09:01, HLA-DPB1*05:01, HLA-DPB1*04:02, HLA-DPB1*04:01, and/or HLA-DPB1 *02:01, based on the base sequences obtained in the above-described manner.

### (2-2-2) PCR method

The alleles according to the present invention can be detected using the PCR method. The PCR method is performed using oligonucleotide primers which only hybridize with the sequences having the alleles according to the present invention or the sequences having other alleles. Further, cDNA derived from genomic DNA or mRNA of the specimen is amplified using this primer set. The specimen includes alleles according to the present invention in the form of homoalleles in a case where a PCR product is generated only by primers for alleles of the present invention, and the specimen includes alleles according to the present invention in the form of heteroalleles in a case where a PCR product is generated by primers for the alleles of the present invention and primers for other alleles. It is shown that the specimen does not have the alleles according to the present invention in a case where a PCR product is generated only by primers for other alleles.

### (2-2-3) PCR-RFLP method

The alleles of the present invention can be detected using restriction fragment length polymorphism (RFLP). First, a region including the alleles of the present invention, which are the detection targets, is amplified by PCR. Next, the PCR product is cleaved by the restriction enzymes suitable for the alleles according to the present invention. The PCR product digested by the restriction enzymes is separated by gel electrophoresis and visualized by ethidium bromide staining. The presence of the alleles of the present invention in the specimen can be detected by comparing the fragment length with the fragment length generated through comparison of molecular weight markers with other alleles, and the alleles of the present invention.

### (2-2-4) Hybridization method

The alleles of the present invention can be detected using hybridization. The hybridization method is a method of determining the presence or absence of the alleles of the present invention based on the properties of genomic DNA or mRNA derived from the specimen hybridizing with DNA molecules (for example, oligonucleotide probes) complementary thereto. The hybridization method can be performed using various techniques for detection and hybridization, for example, known hybridization such as colony hybridization, plaque hybridization, or Southern blotting. Specific procedures of the hybridization method can be referred to in "Molecular Cloning, A laboratory Manual 3rd ed." (Cold Spring Harbor Press (2001): particularly Section 6 and 7), "Current Protocols in Molecular Biology" (John Wiley & Sons (1987 to 1997); particularly Section 6.3 and 6.4), and "DNA Cloning 1: Core Techniques, A Practical Approach 2nd ed." (Oxford University (1995); the hybridization conditions can be particularly referred to in Section 2.10). Further, the hybridization can be detected using a DNA chip. In the method, an oligonucleotide probe specific to the alleles of the present invention is designed and is attached to a solid support for use. Moreover, the hybridization is detected by bringing a DNA sample derived from the specimen into contact with the DNA chip.

### (2-2-5) Other methods

For example, the TaqMan PCR method is a method of concurrently performing detection of SNP and amplification of a region including the SNP using a Taqman probe specific to alleles and a Taq polymerase. The Taqman probe is the oligonucleotide before and after approximately 20 bases in which the 5' end and the 3' end are respectively labeled with a fluorescent material and a quencher and is designed to hybridize with a target SNP site. Taq polymerase has 5'-3' nuclease activity. When a region including target alleles is amplified using a PCR primer designed to amplify the region including target alleles in the presence of the Taqman probe and the Taq polymerase, the Taqman probe hybridizes with the target allele site of template DNA concurrently with the amplification. When an extension reaction from the forward primer side reaches the Taqman probe which has hybridized with template DNA, the fluorescent substance bonded to the 5' end of the Taqman probe is cleaved by 5' nuclease activity of the Taq polymerase. As a result, the released fluorescent substance is no longer affected by the quencher and generates fluorescence. The SNP detection becomes possible through measurement of fluorescence intensity.

As an SNP typing method applying the MALDI-TOF/MS method, a method formed by being combined with the primer extension method can be also exemplified. According to the method, high-through put analysis is possible and the analysis is made by performing steps of 1) PCR, 2) purification of a PCR product, 3) a primer extension reaction, 4) purification of an extension product, 5) mass spectrometry, and 6) genotype determination. First, a region including the target SNP site is amplified from genomic DNA by PCR. The PCR primer is designed so as not to overlap the allele site base. In addition, purification is performed by an enzymatic removal method using exonuclease and shrimp alkaline phosphatase or by using an ethanol precipitation method. Next, the primer extension reaction is performed using a genotyping primer designed such that the 3' end is directly adjacent to the SNP site. The PCR product is denatured at a high temperature and annealed by adding excessive genotyping primers. When ddNTP and the DNA polymerase are added to a reaction system for a thermal cycling reaction, oligomers which are longer than the genotyping primers by a length of one base are generated. The oligomers which are geneated by the extension reaction and are longer than the genotyping primers by a length of one base vary depending on the alleles due to the design of the genotyping primers. The mass spectrometry of the purified extension reaction product is performed and analysis is performed from a mass spectrum.

As the SNP typing method capable of high throughput, a method of applying a single molecule fluorescene analysis method is exemplified. For example, MF20/10S (Olympus) is a system that employs the method. Specifically, the system measures and analyzes the translational diffusion time in one molecule level of fluorescence labeled primers amplified by the PCR method using complementary and non-complementary primers in an ultrafine region of approximately one femtoliter (one quadrillionth liters) using a confocal laser optical system and a high sensitivity photodetector.

In addition, a method using a DNA chip is one of typing methods capable of high throughput. A DNA chip is formed by aligning multiple kinds of DNA probes on a substrate and fixing the probes thereto, and labeled DNA samples are hybridized on a chip and fluorescent signals are detected by a probe.

As the SNP typing method using a gene amplification method other than the PCR method, a Snipper method is exemplified. The method is an SNP typing method that applies a rolling circle amplification (RCA) method, which is a DNA amplification method of synthesizing complementary chain DNA while a DNA polymerase moves on circular single-stranded DNA used as a template. A probe is oligo DNA having a base length of 80 to 90, and the 5' end and the 3' end of the target SNP respectively have complementary sequences having a base length of 10 to 20 in the vicinity of both ends. Further, the probe is designed to be circular by being annealed to the target DNA. Moreover, the 3' end of the probe is designed to be a sequence complementary to the target allele. The probe becomes circular when the 3' end of the probe is completely complementary to the target allele and does not become circular when the 3' end of the probe mismatches the target allele. Moreover, the probe has a backbone sequence having a base length of 40 to 50 and two kinds of RCA amplification primers and complementary seqeuces are included.

As the SNP typing method using the gene amplification method other than the PCR method, a typing method using a UCAN method or an LAMP method is exemplified. The UCAN method is a method that applies an ICAN method which is a gene isothermal amplification method developed by Takara Bio Inc. In the UCAN method, a DNA-RNA-DNA chimeric oligonucleotide (DRD) is used as a primer precursor. The DRD primer precursor is designed such that DNA of the 3' end is modified such that replication of template DNA due to the DNA polymerase does not occur and the RNA portion is bonded to the SNP site. When the DRD primer precursor is incubated with the template, coexisting RNase H cleaves the RNA portion of the pairing DRD primer only in a case where the DRD primer completely matches the template. In this manner, since the 3' end of the primer becomes new by the modified DNA being separated therefrom, the extension reaction using the DNA polymerase proceeds and template DNA is amplified. Meanwhile, in a case where the DRD primer does not match the template DNA, RNase H does not cleave the DRD primer and DNA amplification does not occur. The amplification reaction after the DRD primer precursor which perfectly matches the template DNA is cleaved by RNase H proceeds through an ICAN reaction mechanism.

The LAMP method is a gene isothermal amplification method developed by Eiken Chemical Co., Ltd. and defines six regions (F3c, F2c, and F1c from the 3' end side and B3, B2, and B1 from the 5' end side) of target genes, and amplification is made using four kinds of primers (an FIP primer, an F3 primer, a BIP primer, and a B3 primer) with respect to the six regions. For the purpose of typing, the region between F1 and B1 may be formed of only a target SNP site (one base) and the FIP primer and the BIP primer are designed such that the one base of SNP comes close to the 5' end. In a case of a WT allele, a synthesis reaction of DNA occurs from a dumbbell structure which is a starting point structure of the LAMP method and an amplification reaction continuously proceeds. In a case where the allele is present, the DNA synthesis reaction does not occur from the dumbbell structure and the amplification reaction does not proceed.

The invader method is a method which does not use a nucleic acid amplification method, but uses two kinds of non-fluorescent-labeled probes (an allele probe and an invader probe), one kind of fluorescent-labeled probe (an FRET probe), and cleavase which is an endonuclease. The allele probe are sequences complementary to the template DNA from the SNP site to the 3' end side and has sequences unrelated to the template DNA which is a flap on the 5' side of the probe. The invader probe has complementary sequences to the 5' side from the SNP site of the template DNA, and a base in a portion corresponding to the SNP site is an arbitrary base. The FRET probe has sequences complementary to the flap sequence in the 3' side. The 5' side is labeled with a fluorescent pigment and a quencher, but the FRET probe is designed to form double strands in a molecule and is normally quenched. If these are allowed to react with the template DNA, the 3' end (arbitrary base portion) of the invader probe enters the SNP site when the allele probe forms double strands with template DNA. The cleavase recognizes the structure in which the base has entered and cleaves the flap portion of the allele probe. Next, when the released flap is bonded to the complementary sequence of the FRET probe, the 3' end of the flap enters the double-stranded portion in a molecule of the FRET probe. The cleavase recognizes the structure in which a base of the flap enters this FRET probe and cleaves the fluorescent pigment of the FRET probe, similar to the case of the above-described allele probe and invador probe. Since the fluorescent pigment is separated from the quencher, fluorescence is generated. In a case where the allele probe does not match the allele, the flap is not cleaved because the above-described specific structure, which is recognized by the cleavase, is not formed.

### (2-2-6) Substances used for detection of alleles

Polynucleotides that detect alleles using the method of the present invention will be described below.

In a case where a primer is used for detection of alleles, the primer is designed to be adapted to a region to be amplified and a typing method. For example, it is preferable that the region can be completely amplified, and sequences can be designed based on the sequences in the vicinity of both ends of the region. The method of designing a primer is well-known in the art and a primer which can be used in the present invention is designed to have the length and the base composition (melting temperature), in which specific annealing can be performed, satisfying the conditions in which specific annealing can be performed. The length of the region to be amplified is not limited as long as typing is not disturbed and may be appropriately adjusted according to the detection method. In addition, a part of the region to be amplified includes an allele site, but the position of the site in the region to be amplified is not limited and may be arranged in a suitable position according to the detection method (typing method). For this reason, at the time when a primer is designed, the positional relationship between the primer and the allele site can be freely designed according to the detection method, and the primer can be designed by considering the characteristics of the typing method as long as some regions (for example, the length of continuous 50 bases to the length of continuous 500 bases) of the base sequences shown in SEQ ID NO: 1, 3, 5, 7, and 9 including alleles expected to be detected are hybridized. The length exhibiting the functions as a primer is preferably in a range of 10 bases to 100 bases, typically in a range of 15 bases to 50 bases, and preferably in a range of 15 bases to 30 bases. At the time of designing, it is preferable to confirm the melting temperature (Tm) of the primer which is the temperature at which 50% of arbitrary nucleic acid chains and complementary chains thereof form a hybrid. In order for DNA which becomes the template and a primer to form double strands for annealing, the temperature of the annealing needs to be optimized. Meanwhile, when the temperature is extremely low, a non-specific reaction occurs, which is not preferable. The Tm can be confirmed using known software for designing a primer.

In a case where a probe is used to detect alleles, the probe is designed to recognize allele sites. When the probe is designed, an allele site may be recognized at any location in the probe according to the typing method and may be recognized at the end of the probe depending on the typing method. In a case where a polynucleotide for detection of alleles is used as a probe, the length of the base sequence complementary to genomic DNA is typically in a range of 15 to 200, preferably in a range of 15 bases to 100 bases, and more preferably 15 bases to 50 bases, and the length may be longer or shorter than the above-described range depending on the typing method.

### (2-2-7) Method of detecting alleles preferable for present invention

A preferable method of detecting alleles as the method of the present invention may be a Sequence Specific Oligonucleotide probe (PCR-SSOP) method using PCR, and the alleles of HLA-DPB1 1*09:01, HLA-DPB1 *05:01, HLA-DPB1*04:02, HLA-DPB1*04:01, and/or HLA-DPB1*02:01 can be detected using the method. Specifically, the PCR-SSOP amplifies a region including the alleles of the specimen by PCR using a biotin-labeled primer. The amplified DNA is set as single-stranded DNA and is specifically bonded to a probe which is a specific sequence. For example, since the probe is fixed to microbeads colored by the fluorescent pigment and a fluorescence signal due to binding of fluorescence-labeled streptavidin through biotin from the microbeads to which the amplified DNA is bonded can be obtained, the gene type can be determined from the type of beads to which the amplified DNA is bonded by identifying and detecting the type of the fluorescence signal and the fluorescence caused by binding of the amplified DNA at the same time. Further, commercially available kits may be used for the method. Examples of the kit include xMAP (registered trademark) technology (Luminex Corporation) which is capable of determining multiple polymorphisms at once, but are not limited thereto. The outline of the method of the present invention, which uses the present method will be described below.

First, the amplification reaction in which prepared genomic DNA is used as the template is performed using polynucleotides such as primers or probes which are capable of initially amplifying the region including the alleles of HLA-DPB1 *09:01, HLA-DPB1*05:01, HLA-DPB1*04:02, HLA-DPB1*04:01, and/or HLA-DPB1*02:01, and a nucleic acid fragment having the above-described base sequences is amplified.

The polynucleotides used to detect the alleles can be chemically synthesized using to a known oligonucleotide synthesis method, according to the detection method suitable for a primer or a probe based on the base sequences shown in SEQ ID NOS: 1,3, 5, 7, and 9, and the synthesis can be carried out using a commercially available chemical synthesizer. The polynucleotides can be synthesized by those skilled in the art using a known method based on the base sequences shown in SEQ ID NOS: 1, 3, 5, 7, and 9, complementary chains of these, and position information of the alleles of the present invention. Moreover, in the synthesis of the oligonucleotide, the polynucleotides may be modified using a nucleotide derivative modified by a fluorescent pigment or biotin or the fluorescent pigment or the like may be bonded to the synthesized polynucleotides, and this synthesis method is also known.

Further, the PCR reaction is performed on genomic DNA prepared from the specimen by operating the primers and a thermostable DNA polymerase. The above-described method can be easily performed, by those skilled in the art, by following "Molecular Cloning, A Laboratory Manual 3rd ed." (Cold Spring Harbor Press (2001)) or the like, and the conditions of the PCR reaction of the present invention are, for example, as follows.
Denaturation temperature: 90°C to 100°C
Annealing temperature: 40°C to 70°C
Extension temperature: 60°C to 75°C
The number of cycles described above: approximately 30 times to 50 times

For the purpose of increasing specificity of amplification, the amplification reaction may be performed two times or more using two sets or more of the primers. At this time, the primers used in each of the amplification reactions may be designed to be in the same position or in the inside of the position of the primers used in the initial amplification. In this manner, the nucleic acid fragment of the region including the base sequences of the alleles of the present invention can be specifically amplified using the genomic DNA of the specimen as the template.

Next, after the amplified nucleic acid fragment is purified, base sequences are determined and the determined base sequences are compared to the base sequences of the alleles of the present invention. In this manner, it is possible to determine whether the specimen includes the alleles of the present invention. A known method can be used for purification of the obtained PCR product. Examples of the method include a method of using kits such as Wizard SV Gel and PCR clean-UP System (Promega Corporation), GENECLEAN (Funakoshi Co., Ltd.), QIAquick PCR purification Kits (QIAGEN), and ExoSAP-IT (GE Healthcare Bio-Science), a method of using DEAE-cellulose filter paper, and a method of using a dialysis tube. In a case of using an agarose gel, purification can be performed by performing agarose gel electrophoresis, cutting out a base sequence fragment from the agarose gel, and using Wizard SV Gel and PCR clean-UP System (Promega Corporation), GENECLEAN (Funakoshi Co., Ltd.), or QIAquick Gel extraction Kits (QIAGEN) according to a freeze & squeeze method. Any known methods in the art may be used for sequencing and a direct sequencing method in which the sequence can be determined without cloning an amplified nucleic acid fragment in a vector can be exemplified, but the example is not limited thereto. The sequencing method can be easily performed using commercially available kits such as CEQTMDTCS Quick Start Kit (BECKMAN COULTER Inc.) and BigDye Terminator Cycle Sequencing Ready Reaction Kit ABI310 (Applied Biosystems). In a case where the direct sequencing method is performed, it is preferable to use primers capable of specifically determining base sequences of the region including the alleles of the present invention. A primer set to be used can be designed by a known method.

In this manner, after the base sequences of the region corresponding to the alleles of the present invention in the specimen are determined, these base sequences are compared to the base sequences of the alleles of the present invention.

### (2-3) Process of comparing alleles associated with chronicity of hepatitis B to amino acid sequences corresponding to amino acid sequences of alleles in amino acid sequences obtained from specimen

According to the method of the present invention, the alleles of the present invention can be detected in the protein level. Specifically, the alleles of the present invention can be detected using an antibody that is capable of specifically recognizing DP molecules having the alleles of the present invention. The antibody can be prepared using peptides including any region of the amino acid sequences shown in SEQ ID NOS: 2, 4, 6, 8, and 10 as an antigen according to an immunological method. The method of preparing an antibody and a method of detecting DP molecules, which have the alleles of the present invention, using an antibody can be performed using a known method in the related art.

### (2-4) Determination of resistance or susceptibility to onset of chronicity of hepatitis B or pathological progress

In the present invention, the alleles of HLA-DPB 1*04:02, HLA-DPB1*04:01, and HLA-DPB1*02:01 show that the specimen has resistance to the onset of chronicity of hepatitis B, the alleles of HLA-DPB1 1*05:01 and HLA-DPB1 1*09:01 show that the specimen has susceptibility to the onset of chronicity of hepatitis B, and the allele of HLA-DPB1*02:01 shows that the specimen has resistance to the pathological progress of chronic hepatitis B. In this sense, it can be said that the method of the present invention is a method of inspecting chronic hepatitis B or the pathological progress using the alleles of the present invention.

Further, the alleles having susceptibility and the alleles having resistance to the onset of chronicity of hepatitis B or the pathological progress can be evaluated by means of being used for calculation of diagnostic utility and then analyzing the positive rate of chronicity of hepatitis B or the pathological progress in hepatitis B patients. The "positive rate" related to chronicity of hepatitis B indicates a ratio of patients that have one or more alleles having susceptibility to chronicity of hepatitis B or the like to all the patients or a ratio of patients that have none or only one allele having resistance to chronicity of hepatitis B or the like to all the patients. For example, among 488 Japanese HBV patients described in examples of the present specification, the number of patients that have one or both of HLA-DPB 1*05:01 and HLA-DPB1*09:01 which are alleles having susceptibility to chronicity of hepatitis B is 410 and the number of patients that have neither of them is 78, and thus the positive rate is 84.02%. Further, the number of patients that have none or only one of HLA-DPB1 *04:02, HLA-DPB1 *04:01, and HLA-DPB1*02:01 which are alleles having resistance to chronicity of hepatitis B is 450 and the number of patients that have two of them is 38, and thus the positive rate is 92.21%. Moreover, in regard to the "positive rate" related to the pathological progress of hepatitis B, among 206 cirrhosis and liver cancer patients, the number of patients that have none or only one of HLA-DPB1 1*02:01 which is an allele having resistance to the pathological progress of hepatitis B is 203 and the number of patients that have two of them is 3, and thus the positive rate is 98.5%. Here, the "positive rate" related to the pathological progress of hepatitis B indicates the positive rate related to cirrhosis and liver cancer patients.

The presence or absence of susceptibility to the onset of chronicity of hepatitis B is important information not only for chronic hepatitis B patients but also for non-chronic patients, and this becomes important information related to a treatment method of chronic hepatitis B, selection for treatment medicine, and prevention or onset prevention of chronic hepatitis B. In addition, the presence of alleles of HLA-DPB1*05:01, HLA-DPB1*04:02, HLA-DPB*09:01, HLA-DPB1*04:01, and HLA-DPB1*02:01 may be in the form of homoalleles or heteroalleles because a human has two kinds of genes originating from the father and the mother for each gene.

With respect to humans having a combination of specific resistant alleles and susceptible alleles among resistant alleles having resistance to chronicity of hepatitis B and susceptible alleles having susceptibility to chronicity of hepatitis B, an odds ratio (OR) in a 95% confidence interval of an HBV patient group consisting of 1380 people and a healthy control group consisting of 1225 people was acquired by the present inventors. Here, the odds ratio OR indicates a ratio of odds OsP of an HBV patient group to odds OsC of a healthy control group with respect to a group consisting of humans having a combination of specific alleles. The odds OsP of the HBV patient group was acquired by calculating a ratio of the number of HBV patients having a combination of specific alleles to the number of HBV patients. Further, the odds OsC of the healthy control group was acquired by calculating a ratio of the number of people in the healthy control group who have a combination of specific alleles to the number of people of the healthy control group. Hereinafter, as a specific example of the combination of specific alleles, a group of humans having any one of HLA-DPB1 *04:02, HLA-DPB1*04:01, and HLA-DPB1*02:01 as the resistant alleles and having any one of HLA-DPB1*05:01 and HLA-DPB1*09:01 as the susceptible alleles will be described.

In this specific example, the number of humans having a combination of specific alleles shown in this specific example in a group consisting of 1380 HBV patients is 411. That is, the odds OsP of the HBV patient group with respect to humans having a combination of specific alleles shown in this specific example are 0.42. Moreover, in this specific example, the number of humans having a combination of specific alleles shown in this specific example among a control group consisting of 1225 healthy controls is 477. That is, the odds OsC of the healthy control group with respect to humans having a combination of specific alleles shown in this specific example are 0.63. Accordingly, the odds ratio OR, that is, the ratio of the odds OsP of the HBV patient group to the odds OsC of the healthy control group with respect to humans having a combination of specific alleles shown in this specific example is 0.67.

Here, in a case where the odds ratio OR is less than 1, this means that humans having a combination of specific alleles are resistant to chronicity of hepatitis B. In this specific example, humans having any one of HLA-DPB1 1*04:02, HLA-DPB1*04:01, and HLA-DPB1*02:01 as the resistant alleles and having anyone of HLA-DPB1 1*05:01 and HLA-DPB1*09:01 as the susceptible alleles are resistant to chronicity of hepatitis B.

In other words, it becomes evident that the specimen has resistance to chronicity of hepatitis B in a case where at least HLA-DPB1 alleles have the above-described combinations shown in the specific example when the presence of two kinds of HLA-DPB1 alleles originating from the father and the mother is in the form of heteroalleles of a resistant allele and a susceptible allele.

That is, the present inventors deduced that humans having only one resistant allele and only one susceptible allele are resistant to chronicity of hepatitis B. From this, it can be said that humans having only one resistant allele and only one susceptible allele, that are, two kinds of alleles originating from the father and the mother are resistant to chronicity of hepatitis B. That is, it is possible to determine resistance or susceptibility to the onset of chronicity of hepatitis B or the pathological progress using the alleles of the present invention based on the combination of two kinds of alleles originating from the father and the mother.

### (3) Reagent for detecting predisposition of chronicity of hepatitis B or pathological progress, including alleles of present invention

The predisposition of chronicity of hepatitis B or the pathological progress can be detected using the alleles of the present invention. Therefore, a reagent that detects the above-described alleles is useful as a reagent for inspecting chronicity of hepatitis B or the pathological progress. The reagent can be used to determine the resistance or susceptibility to the onset of chronicity of hepatitis B or the pathological progress. Specifically, in addition to the alleles, various primers, and the probes of the present invention, an antibody which can be specifically bonded to the alleles of the present invention, reagents (for example, deoxynucleotide triphosphate (dNTPs) or a DNA polymerase, a buffer solution, and the like) used when SNP typing is performed at the same time, and positive control, other solvents or solutes can be combined with each other and used as reagents. For example, distilled water, a pH buffer reagent, salts, protein, and a surfactant can be combined with each other.

Moreover, depending on the typing method, some of the polynucleotides for detecting the alleles of the present invention such as probes or primers may have sequences unrelated to the alleles of HLA-DPB1 *09:01, HLA-DPB1 *05:01, HLA-DPB1*04:02, HLA-DPB1*04:01, and/or HLA-DPB1*02:01. Further, the polynucleotides for detecting alleles of the present invention may be chimera of DNA and RNA. Furthermore, the polynucleotides for detecting the alleles of the present invention may be labeled with a fluorescent substance or a binding affinity substance such as biotin or digoxin.

Moreover, the reagent of the present invention may contain a reaction reagent such as a buffer constituting a reaction solution, a dNTP mixture, or enzymes (polymerase or the like) in addition to means for detecting specific alleles. The reaction reagent is a reagent having a label which can be detected by appropriate chemical or physical detection means. Examples of a labeling agent, that uses such a labeling substance, which is used for a measuring method include a fluorescent substance, an enzyme, a radioisotope, and a luminescent substance. An ELISA method using an enzyme for labeling is widely used. Examples of the fluorescent substance include fluorescamine and fluorescein isothiocyanate; examples of the enzyme include peroxidase, alkaline phosphatase, malate dehydrase, α-glucosidase, and α-galactosidase; examples of the radioisotope include 125I, 131I, 3H, and 14C; and examples of the luminescent substance include luciferin, lucigenin, luminol, and a luminol derivative.

Moreover, the optimum conditions for a reaction may be provided for a reaction medium or a buffer solution useful for stabilization of a reaction product substance or a stabilizer of a reaction substance may be contained in the reaction medium.

### (4) Kit for detecting predisposition of chronicity of hepatitis B or pathological progress, including alleles of present invention

In a case where the predisposition of chronicity of hepatitis B or the pathological progress is detected using the alleles of the present invention, special conditions or operations are not required. The detection is performed according to the typical conditions and operations of the respective methods and a suitable measurement system can be constructed by adding slight modifications as needed.

When the reagent of the present invention is made into a kit, it becomes possible to most simply and efficiently perform measurement. When the reagent is made into a kit, it becomes possible to perform efficient quantification without needing specialized analytical equipment, skilled operations, and advanced knowledge in a typical examination room or a laboratory. The contents of the configuration and the form of an assay kit are not particularly limited as long as the predetermined purpose can be achieved. Typically it is configured of instructions for means for detecting the alleles of the present invention, a reaction reagent, a reaction medium which becomes a place where a reaction is carried out, and a base material that provides a place of an assay. Moreover, if desired, matching samples intended to be used for comparison criteria or used to create a calibration curve, a detector, and the like may be included. As detection confirmation means for gene transfer of the present invention, means for detecting the above-described labels, such as a spectrometer, a radiation detector, or a light scattering detector can be exemplified.

### (5) Combination with other alleles

The above-described method of the present invention may be used by being combined with other alleles associated with chronicity of hepatitis B or the pathological progress. The alleles to be used may be alleles related to the alleles of HLA-DPB1*09:01, HLA-DPB1*05:01, HLA-DPB1*04:02, HLA-DPB1*04:01, and/or HLA-DPB1*02:01 or may be alleles in sequences other than the above-described sequences as long as the alleles are associated with chronicity of hepatitis B or the pathological progress. The method of the present invention, in other words, depends on the detection of alleles. Typically, an allele is defined in that the base change is present at a frequency of 1% or greater of the population and an allele of which the base change is present at a frequency of less than 1% is referred to as a rare variant. The alleles to be combined in the present invention may be the ones at a frequency of less than 1% regardless of the presence frequency as alleles in addition to the alleles described above. Moreover, the alleles combined in the present invention may be present in any region of genes associated with chronicity of hepatitis B and the regions may be an exon, an intron, 3'-UTR or 5'-UTR and a region adjacent to 3'-UTR or 5'-UTR, and a promoter region. The polynucleotide for detecting other alleles described above can be prepared by those skilled in the art according to the method described in "(2-2) Detection of alleles" above. Further, the number of alleles is not particularly limited, and there may be substitution, deletion, insertion, or addition of one to several tens of bases. Furthermore, the polymorphism may be in the form of single nucleotide polymorphism (SNP), restriction fragment length polymorphism (RFLP), a variable number of tandem repeat (VNTR), or microsatellite polymorphism.

An allele may be known polymorphism or new polymorphism. In a case where known polymorphism is a target to be detected, polymorphism serving as a candidate to be detected can be selected from known polymorphisms which are open to the public in public databases such as GenBank. Further, the polymorphism may be selected using public data: ENSEMBL (http://www.ensembl.org/) or a plotype can be used. The method of selecting SNP constituting the haplotype and the typing method are as described above. The evaluation of whether the haplotype is associated with chronic hepatitis B or the pathological progress can be performed based on statistical tests as described in examples.

The chronic hepatitis B or the pathological progress can be more rapidly and accurately determined when the alleles of the present invention and other alleles are used in combination, which is preferable in terms that reliability of diagnosis is increased.

Hereinafter, the present invention will be described with reference to examples, but the present invention is not limited to these examples and includes various modification examples.

### [Examples]

### (Detection of alleles of the present invention)

An object of the present example is to detect alleles of HLA-DPB1 *04:02, HLA-DPB1*09:01, HLA-DPB1*05:01, HLA-DPB1*04:01, and HLA-DPB1*02:01 of the present invention.

With respective samples derived from 488 Japanese HBV patients and 464 healthy controls, and 251 Korean HBV patients and 140 healthy controls, preparation was made as follows using a QIAamp DNA Mini kit (QIAGEN). First, after 20 µL of QIAGEN Protease was pipetted into a microtube, 200 µL of each sample was added thereto. Further, Buffer AL was added thereto, the mixture was mixed for 15 seconds and incubated at 56°C for 10 minutes, and then the samples were dissolved therein. Subsequently, the solution attached to the inside of the lid was collected by spinning down the microtube for a few seconds. Further, after 200 µL of ethanol (100%) was added to the sample and vortexed again for 15 seconds, the solution attached to the inside of the lid was collected by spinning down the 1.5 mL microtube for a few seconds. The solution was applied to a QIAamp Mini Spin Column and then centrifuged for 1 minute at 6000 × g. The QIAamp Mini Spin Column was allowed to be open, 500 µL of Buffer AW1 was added thereto, and centrifugation was carried out for 1 minute at 6000 × g. The QIAamp Mini Spin Column was allowed to be open, 500 µL of Buffer AW2 was added thereto, and centrifugation was carried out for 3 minutes at 20000 × g. The QIAamp Mini Spin Column was allowed to be open, and 200 µL of Buffer AE or purified water was added thereto. After incubation at room temperature for 1 minute, centrifugation was carried out for 1 minute at 6000 × g and DNA derived from each specimen was recovered.

Using a LABType SSO HLADPA1/DPB1 kit (One Lambda) or a WAKFlow HLA-DPB1 typing kit (Wakunaga Phamaceutical Co., Ltd.) using a PCR-SSOP method with the prepared DNA sample, four-digit HLA typing was performed. The experiment was performed according to the instructions, and a multiplex measurement system (Luminex Corporation) using xMAP technology was used. Specifically, 24.5 µl of an amplification reagent and 0.5 µL of a DNA polymerase solution were added to 2 µL of the DNA sample, and then a PCR reaction was performed under the following conditions.
Denaturation temperature: 93°C (30 seconds)
Annealing temperature: 60°C (30 seconds)
Extension temperature: 72°C (30 seconds)
The number of cycles described above: 40 times
The reaction was respectively performed at 93°C (3 minutes) before the cycles and at 72°C (5 minutes) after the cycles, and the sample was stored at 4°C after the reaction was finished.

5 µL of amplified DNA after the PCR was finished was added to each well of a 96-well PCR plate, to which 5 µL of a denaturation solution was dispensed, was vortexed, and allowed to stand at room temperature for 5 minutes. The above-described denatured and amplified DNA was added to 25 µL of a hybridization mix reagent into which 20 µL of a hybridization solution, 3 µL of beads mix, and 2 µL of SAPE were mixed, and the solution was vortexed to be stirred as the hybridization mix solution. The hybridization mix solution was set in a thermal cycler whose temperature was set to 55°C, and hybridization was performed for 30 minutes. 75 µL of a washing solution was added to each well, and centrifugation was performed at 1000 × g for 1 minute. Thereafter, the supernatant was removed, and 75 µL of a washing solution was added to each well. The block temperature of Luminex XYP was set to 37°C and measurement was performed using a template file that corresponds to the Lot number of the beads mix. A CSV file of the measurement results was allowed to be open using WAKFlow (registered trademark) Typing Software, and the positivity and negativity of each fluorescent bead were automatically determined based on cut-off values described in the determination table. In the automatic determination, beads showing that the fluorescence intensity is greater than or equal to the cut-off value are determined as positive and beads showing that the fluorescence intensity is less than or equal to the cut-off value are determined as negative, and the genotype of HLAwas determined from the patterns of positivity and negativity of each bead.

The results are listed in Tables 1 and 2.

**[Table 1]**

| Allele | Japanese | | | | | | Korean | | | | | |
|---|---|---|---|---|---|---|---|---|---|---|---|---|
| | HBV patients (2n=976) | | Healthy controls (2n=928) | | Fisher's P* | OR* (95% Cl) | HBV patients (2n = 502) | | Healthy controls (2n = 280) | | Fisher's P* | OR* (95% Cl) |
| | count | % | count | % | | | count | % | count | % | | |
| HLA-DP B1 | | | | | | | | | | | | |
| 02:01 | 182 | 18.6 | 227 | 24.5 | 0.0021 | 0.71 (0.56-0.89) | 96 | 19.1 | 67 | 23.9 | >0.1 | 0.75 (0.52-1.09) |
| 02:02 | 30 | 3.1 | 37 | 4.0 | >0.1 | 0.76 (0.45-1.28) | 20 | 4.0 | 18 | 6.4 | >0.1 | 0.60 (0.30-1.24) |
| 03:01 | 48 | 4.9 | 40 | 4.3 | >0.1 | 1.15 (0.73-1.81) | 10 | 2.0 | 11 | 3.9 | >0.1 | 0.50 (0.19-1.31) |
| 04:01 | 20 | 2.0 | 54 | 5.8 | **2.38x10⁻⁵** | 0.34 (0.19-0.58) | 20 | 4.0 | 11 | 3.9 | >0.1 | 1.01 (0.46-2.38) |
| 04:02 | 38 | 3.9 | 92 | 9.9 | **1.59x10⁻⁷** | 0.37 (0.24-0.55) | 14 | 2.8 | 36 | 12.9 | **1.27**×**10⁻⁷** | 0.19 (0.10-0.38) |
| 05:01 | 456 | 46.7 | 358 | 38.6 | 0.0004 | 1.40 (1.16-1.68) | 270 | 53.8 | 103 | 36.8 | **5.13**×**10⁻⁶** | 2.00 (1.47-2.73) |
| 09:01 | 153 | 15.7 | 81 | 8.7 | **3.70x10⁻⁶** | 1.94 (1.45-2.62) | 20 | 4.0 | 5 | 1.8 | >0.1 | 2.28 (0.82-7.86) |
| 13:01 | 19 | 1.9 | 12 | 1.3 | >0.1 | 1.52 (0.69-3.44) | 37 | 7.4 | 21 | 7.5 | >0.1 | 0.98 (0.55-1.80) |
| 14:01 | 15 | 1.5 | 12 | 1.3 | >0.1 | 1.19 (0.52-2.80) | 6 | 1.2 | 1 | 0.4 | >0.1 | 3.37 (0.41-155.7) |
| 17:01 | - | - | - | - | | | 7 | 1.4 | 5 | 1.8 | >0.1 | 0.78 (0.21-3.14) |
| Others** | 15 | 1.5 | 15 | 1.6 | >0.1 | 0.95 (0.46-1.95) | 2 | 0.4 | 2 | 0.7 | >0.1 | 0.56 (0.08-3.97) |

| | | | | | | | | | | | | |
|---|---|---|---|---|---|---|---|---|---|---|---|---|
| * Fisher's p and OR were calculated based on the presence or absence of specific alleles. ** Alleles with a low frequency of less than 1% in both of HBV patents and healthy controls were classified into "others." | | | | | | | | | | | | |

Table 1 shows a comparison of HLA-DPB1 allele frequencies between HBV patient groups and healthy control groups in Japan and Korea. In this manner, in a case of Japanese people, the alleles of HLA-DPB1 *04:02, HLA-DPB1 *04:01, and HLA-DPB1*02:01 showed that the specimen had resistance to the onset of chronicity of hepatitis B and the alleles of HLA-DPB1 1*05:01 and HLA-DPB1 *09:01 showed that the specimen had susceptibility to the onset of chronicity of hepatitis B. In a case of Korean people, the allele of HLA-DPB 1*04:02 showed that the specimen had resistance to the onset of chronicity of hepatitis B and the allele of HLA-DPB1 1*05:01 showed that the specimen had susceptibility to the onset of chronicity of hepatitis B.

The positive rates of alleles (HLA-DPB 1*05:01 and HLA-DPB1*09:01) having susceptibility to the onset of chronicity of hepatitis B and alleles (HLA-DPB1*04:02, HLA-DPB1*04:01, and HLA-DPB1*02:01) having resistance to the onset of chronicity of hepatitis B, listed in Table 1, to hepatitis B patients were calculated. As a result, in a case where people having susceptible alleles among 488 Japanese HBV patients was calculated, the number of patients having one or both of HLA-DPB 1*05:01 and HLA-DPB1*09:01 was 410 and the number of patients that did not have the alleles was 78. Therefore, the positive rate was 84.02%. Similarly, in a case where people having resistant alleles among 488 Japanese HBV patients was calculated, the number of patients having none or one of HLA-DPB1*04:02, HLA-DPB1*04:01, and HLA-DPB1*02:01 was 450 and the number of patients having any two of them was 38. Therefore, the positive rate was 92.21%.

**[Table 2]**

| HLA-DPB1 allele | Japanese | | | | | | Korean | | | | | |
|---|---|---|---|---|---|---|---|---|---|---|---|---|
| | LC+HCC (2n=412) | | IC+CH (2n=522) | | Fisher's P* | OR* (95% Cl) | HCC (2n=286) | | CH (2n=216) | | Fisher's P* | OR* (95% Cl) |
| | count | % | count | % | | | count | % | count | % | | |
| 02:01 | 54 | 13. 1 | 118 | 22.6 | **0.0002** | 0.52 (0.36-0.74) | 40 | 14.0 | 56 | 25.9 | **0.0009** | 0.47 (0.29-0.75) |
| 02:02 | 17 | 4.1 | 12 | 2.3 | >0.1 | | 10 | 3.5 | 10 | 4.6 | >0.1 | |
| 03:01 | 22 | 5.3 | 24 | 4.6 | >0.1 | | 5 | 1.7 | 5 | 2.3 | >0.1 | |
| 04:01 | 11 | 2.7 | 9 | 1.7 | >0.1 | | 14 | 4.9 | 6 | 2.8 | >0.1 | |
| 04:02 | 13 | 3.2 | 23 | 4.4 | >0.1 | | 6 | 2.1 | 8 | 3.7 | >0.1 | |
| 05:01 | 204 | 49. 5 | 234 | 44.8 | >0.1 | | 16 4 | 57.3 | 106 | 49.1 | 0.0710 | 1.39 (0.96-2.02) |
| 09:01 | 65 | 15. 8 | 79 | 15.1 | >0.1 | | 17 | 5.9 | 3 | 1.4 | 0.0103 | 4.48 (1.27-24.1) |
| 13:01 | 11 | 2.7 | 8 | 1.5 | >0.1 | | 24 | 8.4 | 13 | 6.0 | >0.1 | |
| 14:01 | 7 | 1.7 | 8 | 1.5 | >0.1 | | 1 | 0.3 | 5 | 2.3 | 0.0891 | 0.15 (0.003-1.34) |
| 17:01 | - | | | | | | 4 | 1.4 | 3 | 1.4 | >0.1 | |
| Others ** | 8 | 1.5 | 7 | 1.6 | >0.1 | | 1 | 0.3 | 1 | 0.5 | >0.1 | |

Table 2 shows a comparison of HLA-DPB1 allele frequencies between HBV pathology development groups and chronic hepatitis B groups in Japan and Korea. In this manner, in both of Japanese and Korean people, it was shown that the allele of HLA-DPB1*02:01 had resistance to the pathological progress of chronic hepatitis B.

The positive rates of the allele (HLA-DPB1*02:01) having resistance to the pathological progress of hepatitis B, listed in Table 2, to cirrhosis and liver cancer patients were calculated. As a result, among 206 cirrhosis and liver cancer patients, the number of patients having none or only one of HLA-DPB 1*02:01 was 203 and the number of patients having two of them was 3. Therefore, the positive rate was 98.5%.

### (Detection of alleles of present invention; part 2)

An object of the present example is to clarify that the specimen has resistance or susceptibility to chronicity of hepatitis B in a case where the presence of two kinds of HLA-DPB 1 alleles of the present invention, originating from the father and the mother is in the form of heteroalleles. The genotype of HLA was determined for each sample derived from 1380 Japanese HBV patients and 1225 healthy controls. Further, the procedures of determining the genotype are the same as those for detecting the alleles of the present invention described above and thus the description thereof will not be repeated.

The results are listed in Tables 3 to 5.

**[Table 3]**

| Allele | | Japanese | | | | | | | Allele | | Japanese | | | | | |
|---|---|---|---|---|---|---|---|---|---|---|---|---|---|---|---|---|
| | | HBV patients (2n=976) | | Healthy controls (2n=928) | | X^2 | OR* (95% Cl) | | | | HBV patients (2n=1,784) | | Healthy controls (2n=1522) | | X^2 | OR* (95% Cl) |
| | | count % | | count | % | | | | | | count | % | count | % | | |
| HLA-DPB1 | 01:01 | 0 | 0.0 | 0 | 0.0 | | | | HLA-DP B1 | | | | | | | |
| | 02:01 | 182 | 18.6 | 227 | 24.5 | **0.0020** | 0.71 (0.57-0.88) | | | 01:01 | 1 | 0.1 | 1 | 0.1 | 0.9105 | 0.85 (0.05-13.65) |
| | 02:02 | 30 | 3.1 | 37 | 4.0 | 0.2797 | 0.76 (0.47-1.25) | | | 02:01 | 333 | 18.7 | 368 | 24.2 | **1.11E-04** | 0.72 (0.61-0.85) |
| | 03:01 | 48 | 4.9 | 40 | 4.3 | 0.5279 | 1.15 (0.75-1.76) | | | 02:02 | 47 | 2.6 | 51 | 3.4 | 0.2261 | 0.78 (0.52-1.17) |
| | 04:01 | 20 | 2.0 | 54 | 5.8 | **2.10 ×10⁻⁵** | 0.34 (0.20-0.57) | | | 03:01 | 77 | 4.3 | 68 | 4.5 | 0.8319 | 0.96 (0.69-1.35) |
| | 04:02 | 38 | 3.9 | 92 | 9.9 | **1.59×10⁻⁷** | 0.37 (0.24-0.55) | | | 04:01 | 42 | 2.4 | 83 | 5.5 | **3.22E-06** | 0.42 (0.29-0.61) |
| | 05:01 | 456 | 46.7 | 358 | 38.6 | **0.0003** | 1.40 (1.16-1.68) | | | 04:02 | 91 | 5.1 | 129 | 8.5 | **1.04E-04** | 0.58 (0.44-0.77) |
| | 06:01 | 1 | 0.1 | 6 | 0.6 | 0.0499 | 0.15 (0.02-1.31) | | | 05:01 | 838 | 47.0 | 583 | 38.3 | **5.22E-07** | 1.43 (1.24-1.64) |
| | 09:01 | 153 | 15.7 | 81 | 8.7 | **3.92×10⁻⁶** | 1.94 (1.45-2.62) | | | 06:01 | 9 | 0.5 | 7 | 0.5 | 8.54E-01 | 1.10 (0.41-2.95) |
| | 13:01 | 19 | 1.9 | 12 | 1.3 | 0.2600 | 1.52 (0.73-3.14) | | | 09:01 | 248 | 13.9 | 158 | 10.4 | 2.11E-03 | 1.39 (1.13-1.72) |
| | 14:01 | 15 | 1.5 | 12 | 1.3 | 0.6529 | 1.19 (0.55-2.56) | | | 13:01 | 42 | 2.4 | 27 | 1.8 | 0.2447 | 1.34 (0.82-2.18) |
| | 17:01 | 4 | 0.4 | 0 | 0.0 | 0.0509 | | | | 14:01 | 33 | 1.8 | 30 | 2.0 | 0.7993 | 0.94 (0.57-1.54) |
| | 19:01 | 5 | 0.5 | 7 | 0.8 | 0.5048 | 0.68 (0.21-2.14) | | | 17:01 | 4 | 0.2 | 0 | 0.0 | 0.0645 | |
| | 29:01 | 1 | 0.1 | 0 | 0.0 | 0.0290 | 1.91 | | | 19:01 | 9 | 0.5 | 7 | 0.5 | 0.8540 | 1.10 |
| | 41:01 | 4 | 0.4 | 2 | 0.2 | 0.4495 | (0.35-10.43) | | | 21:01 | 1 | 0.1 | 0 | 0.0 | 0.3556 | (0.41-2.95) |
| | | | | | | | | | | 29:01 | 0 | 0.0 | 2 | 0.1 | 0.1256 | |
| | | | | | | | | | | 36:01 | 2 | 0.1 | 0 | 0.0 | 0.1913 | |
| | | | | | | | | | | 38:01 | 1 | 0.1 | 4 | 0.3 | 0.1273 | 0.21 (0.02-1.91) |
| | | | | | | | | | | 41:01 | 6 | 0.3 | 4 | 0.3 | 0.7013 | 1.28 (0.36-4.55) |

| | | | | | | | | | | | | | | | | |
|---|---|---|---|---|---|---|---|---|---|---|---|---|---|---|---|---|
| 1st set HBV patients (n = 488) 2nd set HBV patients (n = 892) VS. VS. Healthy controls (n = 464) Healthy controls (n = 761) | | | | | | | | | | | | | | | | |

Tables 3 show the holding frequency of a single HLA-DPB1 allele in the Japanese HBV patient group and the Japanese healthy control group. In Tables 3, the left table (1st set) shows the results obtained from the Japanese 488 HBV patients and the Japanese 464 healthy controls and the right table (2nd set) shows results obtained from the Japanese 892 HBV patients and the Japanese 761 healthy controls.

In the 1st set, 15 kinds of HLA-DPB1 alleles were detected, by the present inventors, from the HBV patient group and the healthy control group. The 15 kinds of HLA-DPBalleles were HLA-DPB1*01:01, HLA-DPB*02:01, HLA-DPB1*02:02, HLA-DPB1*03:01, HLA-DPB1*04:01, HLA-DPB1*04:02, HLA-DPB1*05:01, HLA-DPB1*06:01, HLA-DPB1*09:01, HLA-DPB1*13:01, HLA-DPB1*14:01, HLA-DPB1*17:01, HLA-DPB1*19:01, HLA-DPB1*29:01, and HLA-DPB1*41:01. With respect to respective 15 kinds of HLA-DPB 1 alleles, it was counted whether humans in which a certain kind of HLA-DPB allele was detected were included in the HBV patient group or the healthy control group. As an example, among humans in which the allele of HLA-DPB1 *02:01 was detected, 182 people were included in the HBV patient group and 227 people were included in the healthy control group. That is, among humans in which the allele of HLA-DPB1 *02:01 was detected, the number of HBV patients was 182 and the number of the healthy controls was 227.

Further, in the 2nd set, 18 kinds of HLA-DPB 1 alleles were detected, by the present inventors, from the HBV patient group and the healthy control group. The 18 kinds of HLA-DPB 1 alleles were HLA-DPB1*01:01, HLA-DPB*02:01, HLA-DPB1*02:02, HLA-DPB1*03:01, HLA-DPB1*04:01, HLA-DPB1*04:02, HLA-DPB1 *05:01, HLA-DPB1 *06:01, HLA-DPB1 *09:01, HLA-DPB1*13:01, HLA-DPB1*14:01, HLA-DPB1*17:01, HLA-DPB1*19:01, HLA-DPB1*21:01, HLA-DPB1*29:01, HLA-DPB1*36:01, HLA-DPB1*38:01, and HLA-DPB1*41:01. With respect to respective 18 kinds of HLA-DPB1 alleles, it was counted whether humans in which a certain kind of HLA-DPB1 allele was detected were included in the HBV patient group or the healthy control group. As an example, among humans in which the allele of HLA-DPB1 1*02:01 was detected, 333 people were included in the HBV patient group and 368 people were included in the healthy control group. That is, among humans in which the allele of HLA-DPB1*02:01 was detected, the number of HBV patients was 333 and the number of the healthy controls was 368.

Further, with respect to humans in the 1st set and the 2nd set, an odds ratio (OR*) in a 95% confidence interval of the HBV patient group and the healthy control group was acquired by the present inventors. Here, the odds ratio OR* indicates a ratio of odds OsP* of the HBV patient group to odds OsC* of the healthy control group with respect to a group consisting of humans having a specific HLA-DPB1 allele. The odds OsP* of the HBV patient group was acquired by calculating a ratio of the number of HBV patients having the specific HLA-DPB1* allele to the number of HBV patients. Further, the odds OsC* of the healthy control group was acquired by calculating a ratio of the number of people in the healthy control group who have the specific HLA-DPB1* allele to the number of people of the healthy control group. Here, in a case where the odds ratio OR* is less than 1, it can be said that the specimen has resistance to chronicity of hepatitis B. Further, in a case where the odds ratio OR* is 1 or greater, it can be said that the specimen has susceptibility to chronicity of hepatitis B.

In this manner, in the case of Japanese people, the alleles of HLA-DPB1 *02:01, HLA-DPB1*04:01, and HLA-DPB1*04:02 show that the specimen has resistance to chronicity of hepatitis B and the alleles of HLA-DPB 1*05:01 and HLA-DPB1*09:01 show that the specimen has susceptibility to chronicity of hepatitis B.

Moreover, a combination of the above-described HLA-DPB 1 alleles having resistance to chronicity of hepatitis B and the above-described HLA-DPB1 alleles having susceptibility to chronicity of hepatitis B was examined by the present inventors as listed in Tables 4.

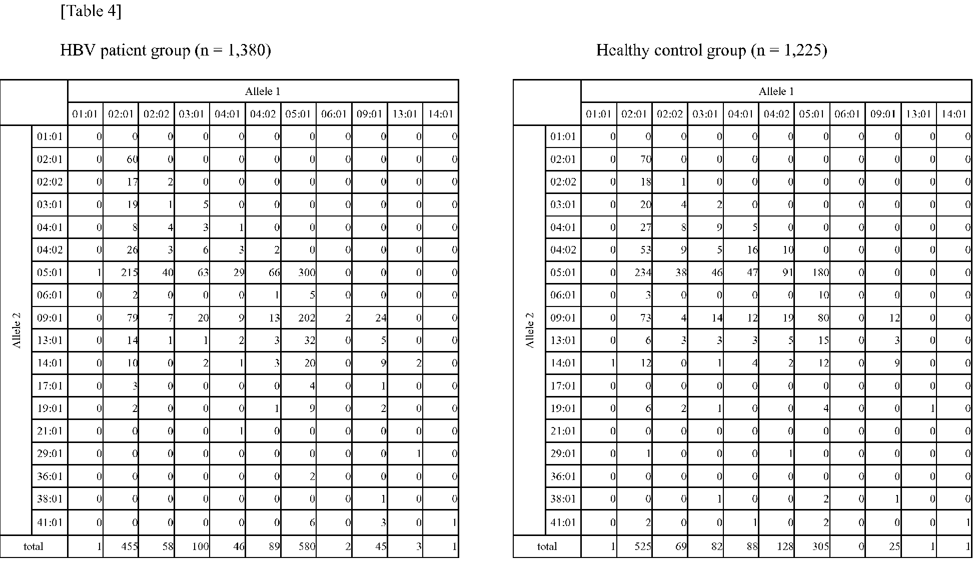

Tables 4 show the holding frequency of a combination of two kinds of HLA-DPB1 alleles originating from the father and the mother, in the Japanese HBV patient group and the Japanese healthy control group. In Tables 4, the left table shows the results obtained from 1380 HBV patients and the right table shows results obtained from 1225 healthy controls. Here, the present inventors focused on the specimen having resistance or susceptibility to chronicity of hepatitis B in a case where the presence of two kinds of HLA-DPB1 alleles originating from the father and the mother is in the form of heteroalleles. Particularly, the present inventors focused on the specimen having resistance or susceptibility to chronicity of hepatitis B in a case of the combination of HLA-DPB1 alleles having resistance to chronicity of hepatitis B and HLA-DPB1 alleles having susceptibility to chronicity of hepatitis B. The results thereof are listed in Table 5.

**[Table 5]**

| | | HBV patients | Healthy controls | Fisher's P | X^2 | OR | 95%Cl | |
|---|---|---|---|---|---|---|---|---|
| | | | | | | | lower | upper |
| 1 | **(0201** or **0401** or **0402)** and **(0501** or **0901)** | 411 | 477 | 1.54E-06 | 1.29E-06 | 0.67 | 0.57 | 0.79 |
| 2 | **0201 and (0501** or **0901)** | 294 | 308 | 2.54E-02 | 2.47E-02 | 0.81 | 0.68 | 0.97 |
| | **0201-0501** | 215 | 235 | 1.94E-02 | 1.86E-02 | 0.78 | 0.64 | 0.96 |
| 3 4 | **0201-0901** | 79 | 73 | 8.67E-01 | 8.08E-01 | 0.96 | 0.69 | 1.33 |
| 5 | 0401 and **(0501** or **0901)** | 38 | 59 | 6.80E-03 | 5.68E-03 | 0.56 | 0.37 | 0.85 |
| 6 | **0401-0501** | 29 | 47 | 1.01E-02 | 8.83E-03 | 0.54 | 0.34 | 0.86 |
| 7 | **0401-0901** | 9 | 12 | 3.86E-01 | 3.53E-01 | 0.66 | 0.28 | 1.58 |
| 8 | **0402** and **(0501** or 0901) | 79 | 110 | 1.86E-03 | 1.46E-03 | 0.62 | 0.46 | 0.83 |
| 9 | **0402-0501** | 66 | 91 | 5.02E-03 | 4.80E-03 | 0.63 | 0.45 | 0.87 |
| 10 | **0402-0901** | 13 | 19 | 2.12E-01 | 1.61E-01 | 0.60 | 0.30 | 1.23 |
| 11 | **(0201** or **0401** or **0402)** and **0501** | 310 | 373 | 6.38E-06 | 5.36E-06 | 0.67 | 0.56 | 0.79 |
| 12 | **(0201** or **0401** or **0402)** and **0901** | 101 | 104 | 2.75E-01 | 2.74E-01 | 0.85 | 0.64 | 1.13 |

Table 5 shows the holding frequency of HLA-DPB1 alleles in a case where the present inventors focused particularly on a combination of HLA-DPB1 alleles having resistance to chronicity of hepatitis B and HLA-DPB1 alleles having susceptibility to chronicity of hepatitis B, among combinations of two kinds of HLA-DPB1 alleles originating from the father and the mother in the Japanese HBV patient group and the Japanese healthy control group.

As an example, among humans having any one of HLA-DPB1*04:02, HLA-DPB1*04:01, and HLA-DPB1*02:01 as the resistant alleles and any one of HLA-DPB1*05:01 and HLA-DPB1*09:01 as the susceptible alleles, 411 people were included in the HBV patient group and 477 people were included in the healthy control group. Among 1380 people of the HBV patient group, the number of humans having a combination of alleles shown in this specific example is 411. Further, among 1225 people of the healthy control group, the number of humans having a combination of specific alleles shown in this specific example is 477. That is, a probability Pp of HBV patients having the combination of alleles shown in this specific example is 0.297 and a probability Ph of healthy controls having the combination of alleles shown in this specific example is 0.388. Therefore, the odds OsP of the HBV patient group with respect to humans having the combination of alleles shown in this specific example are 0.42 and the odds OsC of the healthy control group with respect to humans having the combination of alleles shown in this specific example are 0.63. From this result, with respect to humans having the combination of alleles shown in this specific example, the ratio of the odds OsP of the HBV patient group to the odds OsC of the healthy control group, that is, the odds ratio OR is derived as 0.67.

Hereinbefore, the present invention completed by the present inventors has been described in detail with reference to embodiments, but the present invention is not intended to be limited by the embodiments and various modifications can be made within the range not departing from the scope of the invention.

### Industrial Applicability

The invention is industrially applicable in terms that, by analyzing alleles associated with chronicity of hepatitis B of the present invention with respect to an HBV patient group, it is possible to elucidate a molecular mechanism of chronicity of hepatitis B specialized to Asians including Japanese people and to identify target candidate molecules of drug development. Further, the invention is industrially applicable in terms that, by analyzing alleles with respect to HBV carriers, the carriers can be classified into a group in which chronicity is likely to occur and a group in which chronicity is unlikely to occur so that information useful to determine the subsequent treatment policy can be provided. Furthermore, the invention is industrially applicable in terms that the medical expenses can be reduced by developing test kits including SNP of other immune-related genes in addition to the alleles.

## Claims

1. A method for detecting predisposition for chronicity of hepatitis B and/or the pathological progress, the method comprising:
a) a process of comparing alleles associated with chronicity of hepatitis B and/or the pathological progress with base sequences or amino acid sequences corresponding to the alleles in a specimen;
b) a process of analyzing whether the bases or amino acid residues of sites corresponding to the alleles of the specimen match bases or amino acid residues of the alleles; and
c) a process of specifying whether the hepatitis B of the specimen becomes chronic and/or the pathology is progressed.

2. The method according to claim 1, wherein the alleles associated with chronicity of hepatitis B and/or the pathological progress are susceptible or resistant to chronicity of hepatitis B and/or the pathological progress.

3. The method according to claim 2, wherein a combination of the alleles associated with chronicity of hepatitis B and/or the pathological progress is any of a combination of alleles only having the susceptibility, a combination of alleles only having the resistivity, and a combination of alleles having the susceptibility and alleles having the resistivity.

4. The method according to claim 2 or 3,
wherein the alleles which are susceptible to chronicity of the hepatitis B are HLA-DPB1*05:01 and HLA-DPB1*09:01,
the alleles which are resistant to chronicity of the hepatitis B are HLA-DPB1*04:02, HLA-DPB1*04:01, and HLA-DPB1*02:01, and
the alleles which are resistant to the pathological progress of the hepatitis B are HLA-DPB1*02:01.

5. The method according to any one of claims 2 to 4, wherein the combination of the alleles which are resistant to the chronicity of the hepatitis B is HLA-DPB1*02:01, HLA-DPB1*04:01 or HLA-DPB1*04:02, and HLA-DPB1*05:01 or HLA-DPB1*09:01.

6. A method of inspecting chronicity of hepatitis B and/or the pathological progress using the method according to any one of claims 1 to 5.

7. A reagent for detecting predisposition of chronicity of hepatitis B and/or the pathological progress, the reagent comprising a primer which detects alleles associated with chronicity of hepatitis B and/or the pathological progress.

8. The reagent according to claim 7, wherein the alleles associated with chronicity of the hepatitis B and/or the pathological progress are susceptible or resistant to chronicity of hepatitis B and/or the pathological progress.

9. The reagent according to claim 8, wherein a combination of the alleles associated with chronicity of hepatitis B and/or the pathological progress is any of a combination of alleles only having the susceptibility, a combination of alleles only having the resistivity, and a combination of alleles having the susceptibility and alleles having the resistivity.

10. The reagent according to claim 8 or 9,
wherein the alleles which are susceptible to chronicity of the hepatitis B are HLA-DPB1*05:01 and HLA-DPB1*09:01,
the alleles which are resistant to chronicity of the hepatitis B are HLA-DPB1*04:02, HLA-DPB1*04:01, and HLA-DPB1*02:01, and
the alleles which are resistant to the pathological progress of the hepatitis B are HLA-DPB1*02:01.

11. The reagent according to any one of claims 8 to 10, wherein the combination of the alleles which are resistant to the chronicity of the hepatitis B is HLA-DPB1*02:01, HLA-DPB1*04:01 or HLA-DPB1*04:02, and HLA-DPB1*05:01 or HLA-DPB1*09:01.

12. A kit comprising a reagent which contains alleles associated with chronicity of hepatitis B and/or the pathological progress according to any one of claims 7 to 11 and is used for detecting predisposition of chronicity of hepatitis B and/or the pathological progress.


SEQUENCE LISTING

<110>  Tokyo University
       National Center for Global Health and Medicine

<120>  Method for detecting Predisposition of Chronic hepatitis B

<130>  PC-18967

<160>  11

<170>  PatentIn version 3.5

<210>  1
<211>  917
<212>  DNA
<213>  homo sapiens


<220>
<221>  CDS
<222>  (38)..(814)

<400>  1
tgcagctctt ttcattttgc catccttttc cagctcc atg atg gtt ctg cag gtt        55
                                         Met Met Val Leu Gln Val
                                         1               5

tct gcg gcc ccc cgg aca gtg gct ctg acg gcg tta ctg atg gtg ctg        103
Ser Ala Ala Pro Arg Thr Val Ala Leu Thr Ala Leu Leu Met Val Leu
            10                  15                  20

ctc aca tct gtg gtc cag ggc agg gcc act cca gag aat tac ctt ttc        151
Leu Thr Ser Val Val Gln Gly Arg Ala Thr Pro Glu Asn Tyr Leu Phe
        25                  30                  35

cag gga cgg cag gaa tgc tac gcg ttt aat ggg aca cag cgc ttc ctg        199
Gln Gly Arg Gln Glu Cys Tyr Ala Phe Asn Gly Thr Gln Arg Phe Leu
    40                  45                  50

gag aga tac atc tac aac cgg gag gag ctc gtg cgc ttc gac agc gac        247
Glu Arg Tyr Ile Tyr Asn Arg Glu Glu Leu Val Arg Phe Asp Ser Asp
55                  60                  65                  70

gtg ggg gag ttc cgg gcg gtg acg gag ctg ggg cgg cct gag gcg gag        295
Val Gly Glu Phe Arg Ala Val Thr Glu Leu Gly Arg Pro Glu Ala Glu
                75                  80                  85

tac tgg aac agc cag aag gac atc ctg gag gag aag cgg gca gtg ccg        343
Tyr Trp Asn Ser Gln Lys Asp Ile Leu Glu Glu Lys Arg Ala Val Pro
            90                  95                  100

gac agg atg tgc aga cac aac tac gag ctg gac gag gcc gtg acc ctg        391
Asp Arg Met Cys Arg His Asn Tyr Glu Leu Asp Glu Ala Val Thr Leu
        105                 110                 115

cag cgc cga gtc cag cct aag gtg aac gtt tcc ccc tcc aag aag ggg        439
Gln Arg Arg Val Gln Pro Lys Val Asn Val Ser Pro Ser Lys Lys Gly
    120                 125                 130

ccc ctg cag cac cac aac ctg ctt gtc tgc cac gtg aca gat ttc tac        487
Pro Leu Gln His His Asn Leu Leu Val Cys His Val Thr Asp Phe Tyr

135                 140                 145                 150

cca ggc agc att caa gtc cga tgg ttc ctg aat gga cag gag gaa aca         535
Pro Gly Ser Ile Gln Val Arg Trp Phe Leu Asn Gly Gln Glu Glu Thr
            155                 160                 165